# EUROPEAN PATENT APPLICATION

(11) **EP 4 636 782 A1**
(43) Date of publication of application: **22.10.2025**
(21) Application number: 24170847.8
(22) Date of filing: 17.04.2024
(51) Int. Cl.: G16H 50/30

(54) **COMPUTER-IMPLEMENTED METHOD FOR BIOLOGICAL AGE ESTIMATION**

(71) Applicant: Holland & Barrett International, Nuneaton Warwickshire CV10 7RH (GB)
(72) Inventor: HOWARD, Amanda, Nuneaton, CV10 7RH (GB); LEGGATE, Charles, Nuneaton, CV10 7RH (GB); LOGAN, Christopher, Nuneaton, CV10 7RH (GB); GORODI, Leah, Nuneaton, CV10 7RH (GB); GRAY, Augusta, Nuneaton, CV10 7RH (GB); DOMINICI, Cecilia, Nuneaton, CV10 7RH (GB); PATTINSON, Colin, Nuneaton, CV10 7RH (GB); GLEESON, Darcy, Nuneaton, CV10 7RH (GB); SHAW, Alexander, Nuneaton, CV10 7RH (GB); KUKADIA, Nish, Nuneaton, CV10 7RH (GB); RAJAH, Tamara, Nuneaton, CV10 7RH (GB)
(74) Representative: Appleyard Lees IP LLP

(57) **Abstract**

The subject-matter of the present disclosure relates to a method of recommending an action for a user of an application to take to make a health improvement. The computer-implemented method comprises: receiving a plurality of user inputs responding to queries about a health of the user; generating a biological age based on the user inputs; displaying at least one recommendation to the user for managing the biological age; and receiving at least one further user input to make an action in response to the at least one recommendation.

## Description

### FIELD

The subject-matter of the present disclosure relates to computer-implemented methods and in particular methods which provide guidance to a user to take an action to improve their health.

### BACKGROUND

Mobile applications exist which can help a user track various health related metrics. Tracking these metrics can be done manually, e.g. a user inputting their blood pressure readings, or automatically, e.g. step count from a wearable device. If a user wishes to improve any of these metrics, it is necessary for them to perform certain actions, e.g. do exercise, eat more healthily, take health supplements, etc.

It is an aim of the present invention to improve on the prior art.

### SUMMARY

According to an aspect of the present disclosure, there is provided a computer-implemented method of recommending an action for a user of an application to take to make a health improvement, the computer-implemented method comprising: receiving a plurality of user inputs responding to queries about a health of the user; generating a biological age based on the user inputs; displaying at least one recommendation to the user for managing the biological age; and receiving at least one further user input to make an action in response to the at least one recommendation.

The above aspect of the present disclosure is advantageous over the prior art as it enables the information and products related to improve certain metrics to be located in one place such that the user does not need to find the sources themselves. This is beneficial over the prior art as the information and products related to improve those metrics are in a myriad of different sources such that the user has to find those sources themselves. In addition, using this method, the user is provided with guidance regarding which metrics to focus on improving in order to improve their overall health. E.g. the user may be interested in increasing their number of steps per day, when their efforts would be better served improving their sleep.

In an embodiment, generating the biological age based on the user inputs comprises calculating a biological age score and a plurality of wellness scores based on the user inputs.

In an embodiment, the wellness scores includes at least one of: a sleep quality score, a nutrition intake score, fitness level score, and emotional wellbeing score.

This is advantageous as it allows the user to acquire a more granular breakdown of contributing factors to their biological age.

In an embodiment, displaying at least one recommendation to the user for managing the biological age comprises a goal of a plurality of goals intended to improve at least one of the plurality of wellness scores.

In an embodiment, receiving additional inputs to track performance against the goal; and updating the biological age based on the additional inputs.

In an embodiment, the additional inputs include at least one of an additional user input, and sensor data.

In an embodiment, the sensor data includes at least one of: a step count, exercise minutes, calorie burn estimation, sleep tracking, pulse rate, and blood oxygenation levels.

In an embodiment, the at least one recommendation is at least one of: a product recommendation; and a test kit.

In an embodiment, receiving at least one further user input to make an action in response to the at least one recommendation comprises at least one of: purchasing the product recommendation; and purchasing the test kit.

In an embodiment, receiving a test input describing results from taking a test form the test kit; and updating the biological age based on the test input.

In an embodiment, the at least one recommendation is an article of a plurality of articles.

In an embodiment, receiving at least one further user input to make an action in response to the at least one recommendation comprises selecting the article to view.

In an embodiment, the computer-implemented method further comprises: displaying, using a coach function, a log tracking the at least one further user input.

According to an aspect there is provided a transitory, or non-transitory, computer-readable media having instructions stored thereon that when executed by at least one processor causes the at least one processor to perform the computer-implemented method of any preceding aspect or embodiments.

### BRIEF DESCRIPTION OF DRAWINGS

The subject-matter of the present disclosure is best described with reference to the accompanying figures, in which:
Figure 1 shows a block diagram of a system architecture, according to at least one embodiment;
Figure 2 shows a screen shot of a mobile app from Figure 1 displaying an example question for the health questionnaire;
Figure 3 shows a screen shot of a mobile app from Figure 1 displaying a biological age of a user;
Figure 4 shows a screen shot of a mobile app from Figure 1 displaying a wellness scores of a user;
Figure 5 shows a screen shot of a mobile app from Figure 1 displaying a detailed view of a sleep quality wellness score and the recommended goals;
Figure 6 shows a screen shot display a user's goals in progress and all available goals on a mobile app form Figure 1;
Figure 7 shows a screen shot of a mobile app from Figure 1 displaying recommended products;
Figure 8 shows a screen shot of a mobile app from Figure 1 displaying recommended articles and health tests;
Figure 9 shows a screen shot of a mobile app from Figure 1 displaying results of health tests;
Figure 10 shows a screen shot of a mobile app from Figure 1 displaying a coaching function; and
Figure 11 shows a schematic diagram of a mobile device for running the mobile app from Figure 1.

### DESCRIPTION OF EMBODIMENTS

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. Any reference signs in the claims should not be construed as limiting the scope.

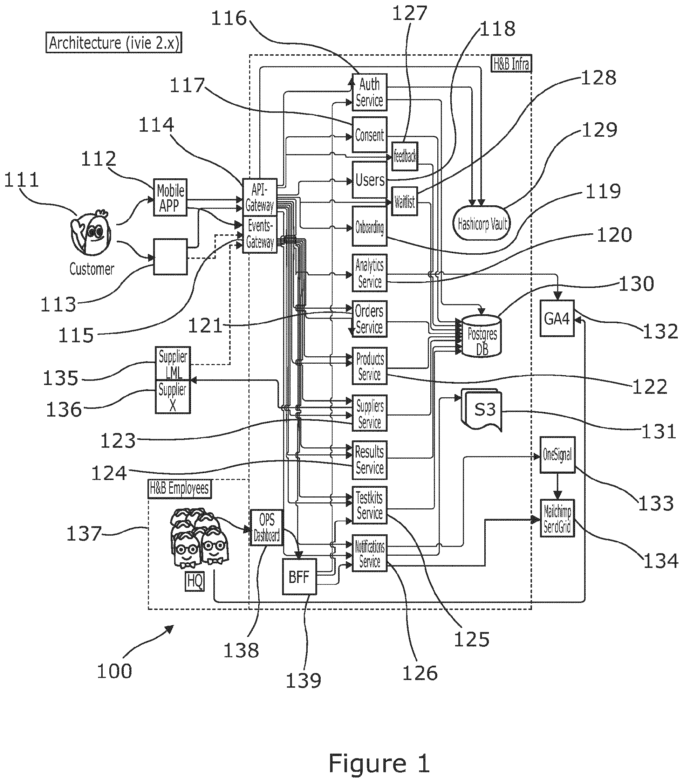

With reference to Fig. 1 there is provided a system architecture 100 comprising a front end and a back end. The front end includes a mobile app 112 and/or a website 113. A user 111 interacts with the system architecture via the mobile app 112. The user may also interact with the system architecture via the website 113. The system architecture 100 includes an application program interface, API, gateway 114 and an events gateway 115. The API Gateway 114 and the Event Gateway 115 link the font end with the back end allowing a user to access functionality managed in the back end.

The mobile app 112 or website 113 may allow the user to interact with an API-Gateway 114 and an Event-Gateway 115. The back end includes a plurality of APIs. The API-Gateway 114 may allow the user 111 to access various The APIs include an authentication service API 116, a consent API 117, a user's API 118, an onboarding API 119, an analytics service API 120, an orders service API 121, a products service API 122, a suppliers service API 123, a results service API 124, a testkits service API 125, a notifications service API 126, a feedback API 127 and a waitlist API 128. The API Gateway 114 allows a user 111 to access at least some of the plurality of APIs. The orders service API 121, the products service API 122, the suppliers service API 123, the results service API 124, the testkits service API 125 and the notifications service API 126 may also be accessed by the Event-Gateway 115.

Data from the API-Gateway 114 and authentication service 116 may be stored in a Hashicorp Vault 129.

Data from the authentication service API 116, the consent API 117, the orders service API 121, the products service API 122, the suppliers service API 123, the results service API 124, the testkits service API 125, the feedback API 127 and the waitlist API 128 may be stored in a database (DB) 130. The DB 130 may be a postgres DB.

The analytics service API 120 may send data to an analytics platform 132 which generates analytics. The analytics platform may be Google (RTM) Analytics, GA4.

The notification services API 126 may transfer data to various notification platforms. The notification platforms include Amazon (RTM) S3 131, OneSignal (RTM) 133, and MailChimp (RTM) SendGrid 134. Other notification platforms may also be used.

The suppliers service API may transfer data to third party testing partner (Supplier LML) 135.

Employees 137 may access an operations (OPS) Dashboard 138 which connects to binary interchange file format (BFF) 139. The BFF 139 may connect to the notifications service API 126, the testkits service API 125 and the authentication service API 116. The OPS Dashboard 138 may also connect to the notifications service API 126 and the API-Gateway 114.

When first interacting with the application through the API-Gateway 114, the user 111 may access the authentication service 116. The authentication service 116 allows the user 111 to register an account. For example, the user 111 may register an account with an email address. The user 111 may also be required to consent to the terms and conditions of the application via a consent module 117.

Upon authenticating an account, the user 111 may set up a user profile through the user's module 118 and the onboarding module 119. These modules allow the user to enter information such as, but not limited to, their name(s), age, date of birth and nationality.

The system architecture 100 may be used to perform various computer implemented methods. For example, the system architecture 100 may be used to perform a computer-implemented method of recommending an action for a user of an application to take to make a health improvement. The computer-implemented method comprises receiving a plurality of user inputs responding to queries about a health of the user.

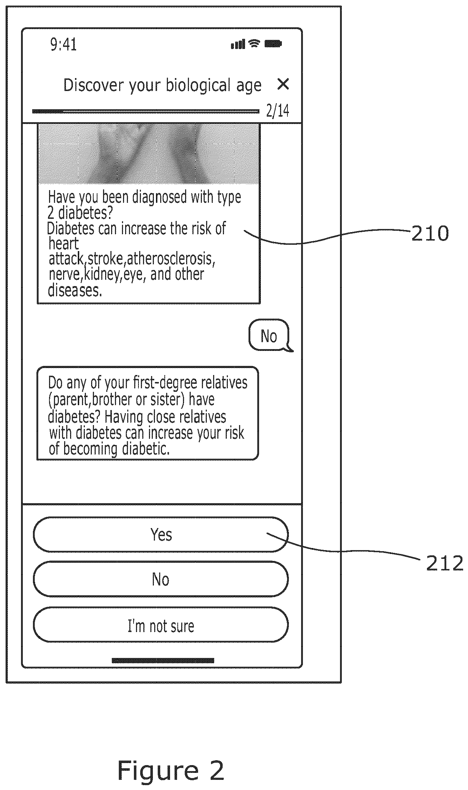

With reference to Figure 2, upon setting up their profile, a user 111 may fill in a health questionnaire including a plurality of questions 210. The health questionnaire may comprise questions relating to the user's 111 sleeping habits, nutritional habits, fitness habits and emotional wellbeing. The user may input the user inputs by selecting one of a plurality of proposed answers 212. For example, the question 210 may be "Have you been diagnosed with type 2 diabetes?" and the answers 212 may include "Yes", "No", "I'm not sure".

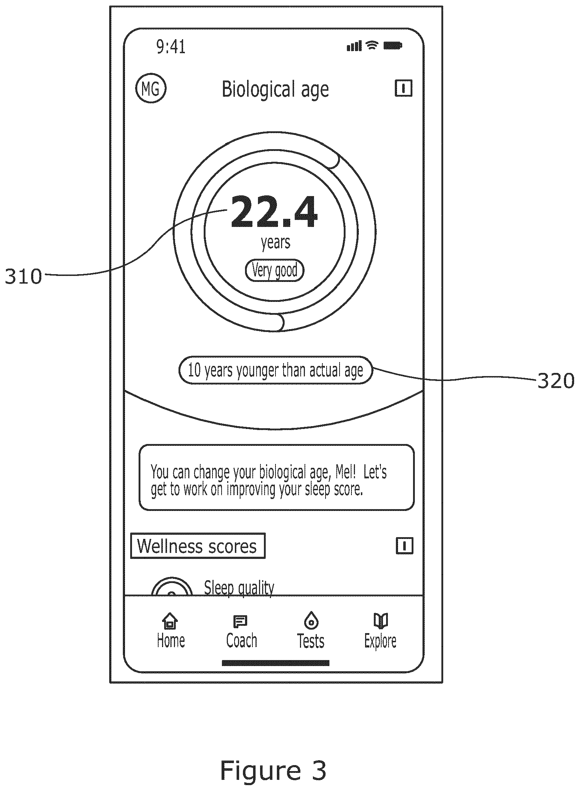

With reference to Figure 3, the computer-implemented method comprises generating a biological age based on the user inputs. For example, the user's inputs to a health questionnaire may be used to generate a biological age. The biological age may be generated by an external source.

The generated biological age may include a biological age score 310 may be displayed to the user of the app. The application may also display the difference between the generated biological age and the user's 111 actual age as seen in reference number 320.

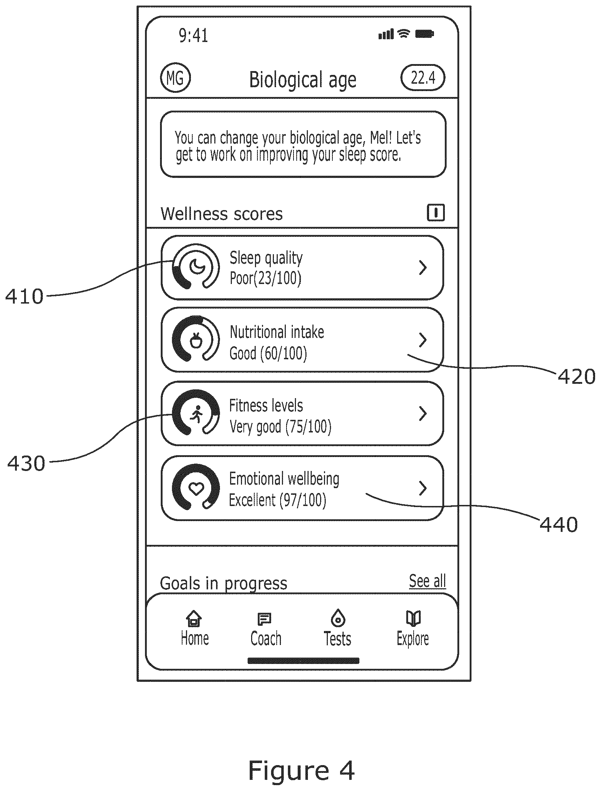

With reference to Figure 4, generating the biological age based on the user inputs comprises calculating the biological age score 310 (Figure 3) and a plurality of wellness scores based on the user inputs. The biological age may be broken down into a number of different categories. For example, the biological age may depend on the following categories; sleep quality, nutritional intake, fitness levels, and emotional wellbeing. Each wellness score for each of these categories may be displayed to the user. The wellness scores may include a sleep quality score 410, a nutritional intake score 420, a fitness levels score 430 and an emotional wellbeing score 440. The wellness scores are generated from the questionnaire used to generate the biological age of the user 111. In this way, the wellness scores includes at least one of: a sleep quality score, a nutrition intake score, fitness level score, and emotional wellbeing score.

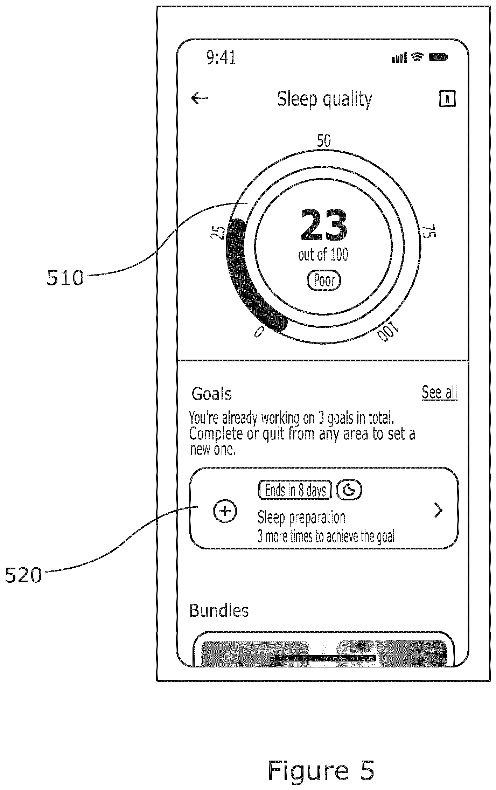

With reference to Figure 5, the user 111 of the app now has the information as to which areas of their wellness they need to improve in order to improve their overall biological age score. The user may be shown more information about their wellness by tapping on the specific wellness score. The information may include a dial 510 indicating the individual wellness score out of 100, for example.

The computer-implemented method comprises displaying at least one recommendation 520 to the user for managing the biological age. Displaying at least one recommendation to the user for managing the biological age comprises a goal 520 of a plurality of goals intended to improve at least one of the plurality of wellness scores. The goals recommended to the user may comprise of tasks that the user completes over a set period. The plurality of goals may include eating more of a certain type of food, achieving a certain number of steps a day, partaking in a certain exercise, e.g. yoga or cycling, sleep duration goals, mindfulness or meditation session goals, etc. As described below, tracking performance against the goals may be done by a user manually inputting information through the app or the app pulling information such as step count from an exercise application, e.g. Apple Health.

The computer-implemented method includes receiving at least one further user input to make an action in response to the at least one recommendation. As the wellness scores affect the biological age of the user, the user's 111 biological age may be adjusted based on the completion of goals that may be recommended to the user. The user may therefore make an action in response to the recommendation of a goal. For example, the action may be to select a goal of the plurality of goals. The selected goal will be visible in the app and the performance against the goal will be displayed.

The goals 520 may be recommended to the user 111 based on their wellness score. For example, the goal recommended to the user on the sleep quality wellness score page relates to improving the sleep quality wellness score. In another example, when on the emotional wellbeing score page, the recommended goals may relate to improving their emotional wellbeing score.

The computer-implemented method comprises receiving additional inputs to track performance against the goal and updating the biological age based on the additional inputs. The additional inputs include at least one of an additional user input, and sensor data.

For example, one goal may be to eat more fibre which would improve the user's 111 nutritional intake score 420. In this example, the user would be required to provide an additional user input to the application such as the user logging in the app every day that the amount of fibre they have consumed that day. If the user completes this goal, then their nutritional intake score 420 may be improved, and as such their biological age may be updated. In order for the biological age to be updated in this scenario, the user will be asked to update the relevant questions around completion of a goal after which biological age and wellness scores will be recalculated. Another example of additional user inputs may be the user logging the number of hours sleep they have had.

Sensor data includes at least one of: a step count, exercise minutes, calorie burn estimation, sleep tracking, pulse rate, and blood oxygenation levels. The sensor data that is recorded in third party applications such as step counting apps, heart rate monitoring apps and sleep logging apps, may be integrated into the computer implemented method such that sensor data may automatically be used as additional inputs to track the user's 111 performance against the goal. For example, if the user has a goal set to reach a certain number of steps per day, the steps counted in the integrated app may contribute to the step goal. The sensor data may be derived from sensors integrated into a device running the app or a separate wearable device, e.g. a smart watch. The sensors may include sensors such as a gyroscope, an accelerometer, a blood oxygen sensor, etc. For goals which are automatically updated using sensor information, the biological age and wellness scores may be updated automatically.

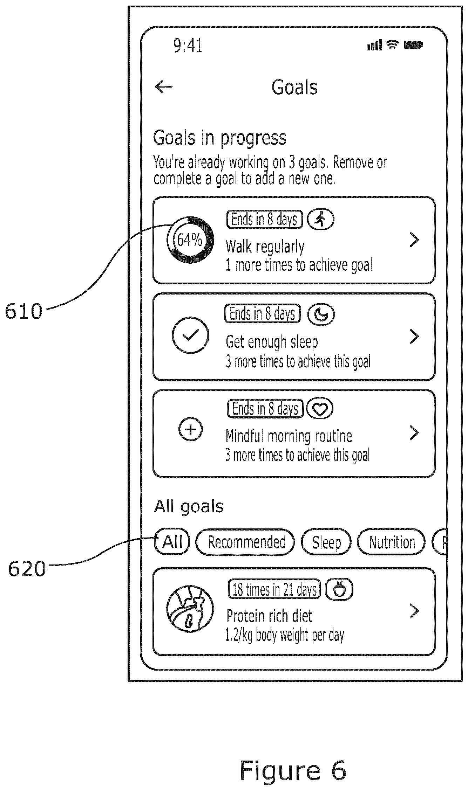

With reference to Figure 6, the app may display the goals 620 the user currently has in progress 610 and all available goals 620. The user may select goals to complete from a predefined list, or may create their own goals. The user may select one of their current goals to log data in the form of an additional user input or they may select one of their current goals to view their progress.

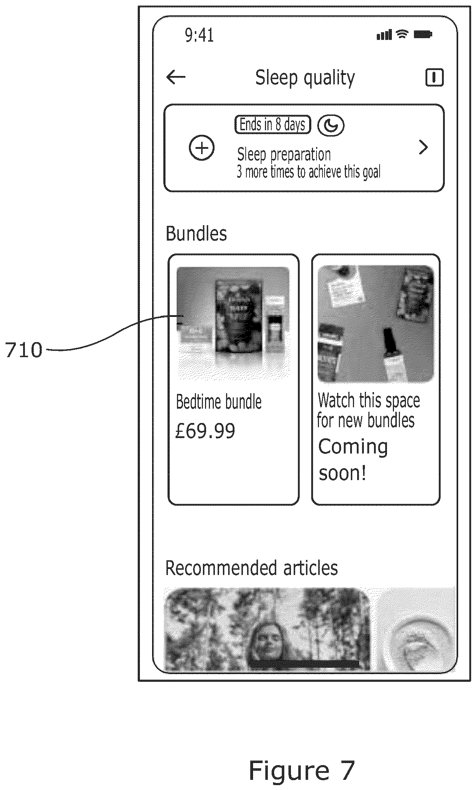

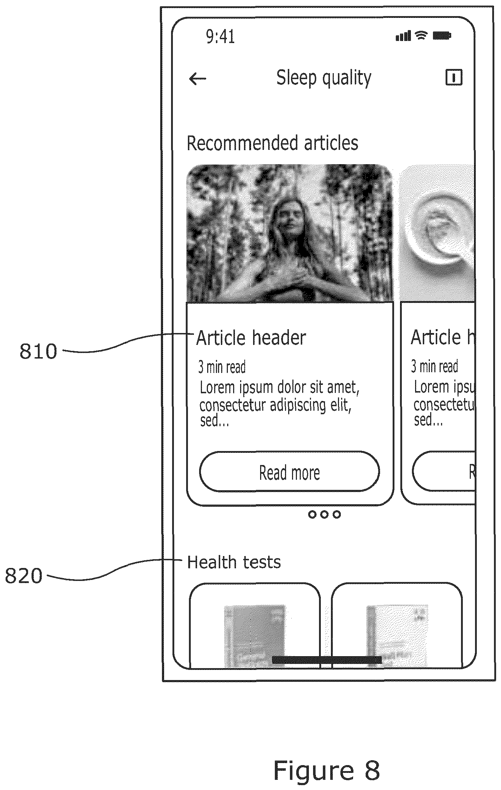

With reference to Figures 7 and 8, the computer-implemented method recommends at least one of a product 710 and a test kit 820. The test kit may also be a called a health test product. Products may recommended to improve a particular wellness score, and in turn improve the user's 111 biological age.

With reference to Figure 7, a product bundle 710 may be recommended to improve the user's 111 nutritional intake.

With reference to Figure 1, the user 111 can access the order service module 121 through the API-Gateway 112. The order service allows the user to order products from the recommendations. For example, the recommendation may show a product and when the user 111 clicks on this product or selects the product, they may be linked through to a website that allows them to purchase the product. The user may therefore make an action in response to the recommendation of a product. The user may purchase a product through accessing the order service API 121 and products service API 122.

In this way, the action is to purchase a health kit (or product). Upon selecting a health test the user may be directed to a website where they can purchase a recommended test kit. As such, receiving at least one further user input to make an action in response to the at least one recommendation comprises at least one of: purchasing the product recommendation; and purchasing the test kit.

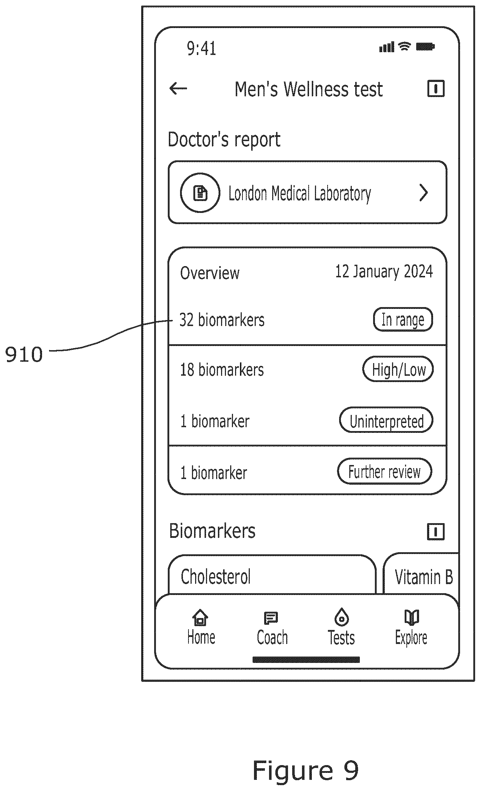

With reference to Figure 9, the computer-implemented method further comprises receiving a test input describing results from taking a test form the test kit; and updating the biological age based on the test input. The application may allow users to input test results 910 that have been purchased through the app, or from a third party. The user may scan a barcode that comes with the testing kit to input their results or input a code that comes with the testing kit. The user may manually input their test results. The test results may also come with a doctor's report and a breakdown of specific biomarkers of the test to see whether they fall in a normal range for that specific user. The user 111 may purchase a test kit/health test/health kit through the testkits service API 123 and receive or view their results through the results service API 124.

With further reference to Figure 8, at least one recommendation is an article of a plurality of articles 810. In this way, receiving at least one further user input to make an action in response to the at least one recommendation comprises selecting the article to view. Articles may relate to a user's 111 wellness score and biological age. For example, if they have a low emotional wellbeing score then the recommended articles may include articles related to mental health. Articles may contain tips and tricks on how to improve a specific wellness score and may contain informative information and videos.

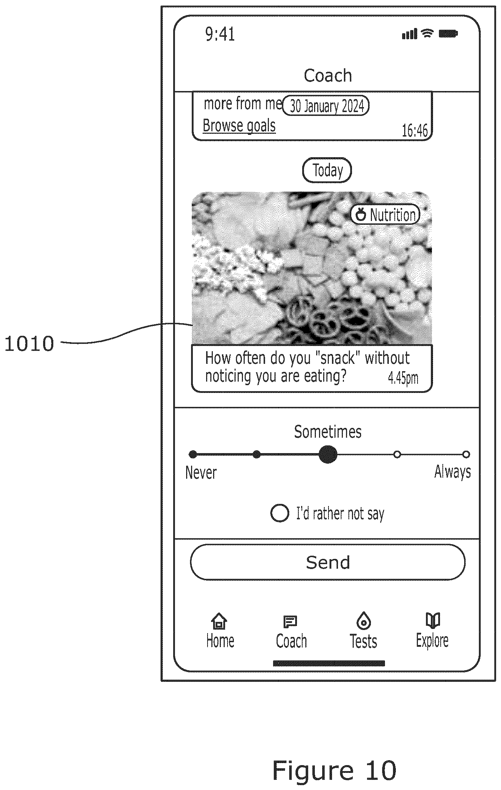

With reference to Figure 10, the computer-implemented method further comprises displaying, using a coach function, a log 1010 tracking the at least one further user input. For example, the coach may send a notification to the user reminding them that they have set a goal. The coach function may also push articles, products, goals and health kits to the user 111. The coach app may use the notifications service API 126. The user 111 may interact with the coach by answering prompts received from the coach function of the app. For example, the coach function may ask the user 111 "how often do you snack without noticing that you are eating" and the user may respond with an answer. Based on the user's 111 answer, the coach app may then provide the user 111 with recommendations such as goals, products, health kits and articles.

The app may also include features such as booking appointments. For example, booking an appointment with a health professional. The app may also include challenges. For example, a challenge may be "March Walk 200k challenge" whereby all users of the app can register for the challenge and will attempt to walk 200k steps in the month of March. The challenges may show a leaderboard with all user's who have joined the challenge.

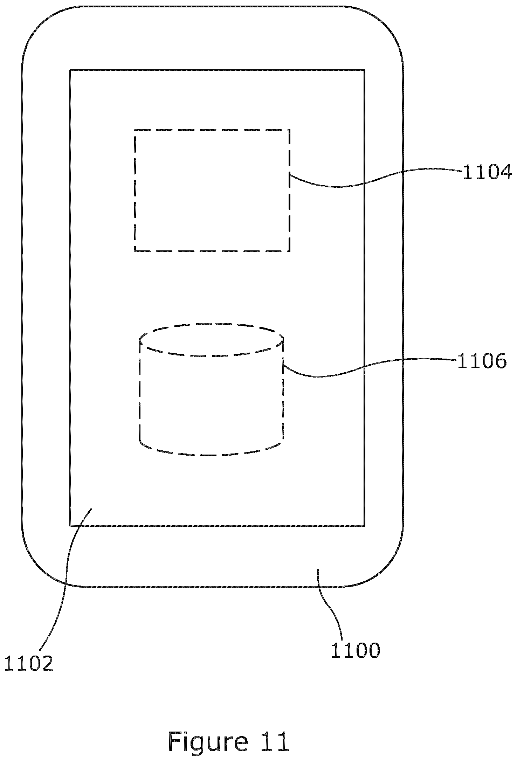

With reference to Figure 11, the app 112 (Figure 1) may be implemented on a mobile device 1100, e.g. a smart phone. The smart phone includes a display 1102, which may provide both a display function and also a user input function. For example, the display 1102 may be a touch screen. The smart phone may also comprise a processor 1104 and storage 1106. The storage may be non-transitory computer readable media having instructions stored thereon that when executed by the processor 1104 causes the at least one processor to perform the computer-implemented method described above. When installing the app 112 into the storage, the instruction may be transitory, e.g. a download.

## Claims

1. A computer-implemented method of recommending an action for a user of an application to take to make a health improvement, the computer-implemented method comprising:
receiving a plurality of user inputs responding to queries about a health of the user;
generating a biological age based on the user inputs;
displaying at least one recommendation to the user for managing the biological age; and
receiving at least one further user input to make an action in response to the at least one recommendation.

2. The computer-implemented method of Claim 1, wherein generating the biological age based on the user inputs comprises calculating a biological age score and a plurality of wellness scores based on the user inputs.

3. The computer-implemented method of Claim 2, wherein the wellness scores includes at least one of: a sleep quality score, a nutrition intake score, fitness level score, and emotional wellbeing score.

4. The computer-implemented method of Claim 2 or Claim 3, wherein displaying at least one recommendation to the user for managing the biological age comprises a goal of a plurality of goals intended to improve at least one of the plurality of wellness scores.

5. The computer-implemented method of Claim 4, further comprising:
receiving additional inputs to track performance against the goal; and
updating the biological age based on the additional inputs.

6. The computer-implemented method of Claim 5, wherein the additional inputs include at least one of an additional user input, and sensor data.

7. The computer-implemented method of Claim 6, wherein the sensor data includes at least one of: a step count, exercise minutes, calorie burn estimation, sleep tracking, pulse rate, and blood oxygenation levels.

8. The computer-implemented method of any preceding claim, wherein the at least one recommendation is at least one of: a product recommendation; and a test kit.

9. The computer-implemented method of Claim 8, wherein receiving at least one further user input to make an action in response to the at least one recommendation comprises at least one of: purchasing the product recommendation; and purchasing the test kit.

10. The computer-implemented method Claim 9, further comprising:
receiving a test input describing results from taking a test form the test kit; and
updating the biological age based on the test input.

11. The computer-implemented method of any preceding claim, wherein the at least one recommendation is an article of a plurality of articles.

12. The computer-implemented method of Claim 11, wherein receiving at least one further user input to make an action in response to the at least one recommendation comprises selecting the article to view.

13. The computer-implemented method of any preceding claim, further comprising:
displaying, using a coach function, a log tracking the at least one further user input.

14. A transitory, or non-transitory, computer-readable media having instructions stored thereon that when executed by at least one processor causes the at least one processor to perform the computer-implemented method of any preceding claim.
